# EUROPEAN PATENT SPECIFICATION

(11) **EP 1 966 136 B1**
(45) Date of publication and mention of the grant of the patent: **26.02.2014**
(21) Application number: 06831515.9
(22) Date of filing: 18.12.2006
(51) Int. Cl.: C07D 211/02, C07D 211/32

(54) **NOVEL PROCESS FOR PRODUCTION OF HIGHLY PURE POLYMORPH (I) DONEPEZIL HYDROCHLORIDE**
NEUES VERFAHREN ZUR HERSTELLUNG VON HOCHREINEM DONEPEZILHYDROCHLORID POLYMORPH(I)
NOUVEAU PROCÉDÉ POUR LA PRODUCTION DE CHLORHYDRATE DE DONÉPÉZIL POLYMORPHE (I) EXTRÊMEMENT PUR

(30) Priority: 20.12.2005 HU 0501167
(43) Date of publication of application: 10.09.2008
(73) Proprietor: Richter Gedeon Nyrt., 1103 Budapest (HU)
(72) Inventor: NEU, József, H-1133 Budapest (HU); GREINER, István, H-1021 Budapest (HU); CSABAI, János, H-1108 Budapest (HU); GARADNAY, Sándor, H-2500 Esztergom (HU)
(74) Representative: Vossius & Partner
(86) International application number: PCT/HU2006/000115
(87) International publication number: WO 2007/072087

(56) References cited:
- WO-A-97/46526
- WO-A-97/46527
- WO-A-2004/082685
- WO-A-2005/044805
- CN-A- 1 524 851

## Description

### FIELD OF THE INVENTION

This invention relates to a new process for the preparation of highly pure donepezil hydrochloride, *i.e.*, 1-benzyl-4-[(5,6-dimethoxy-1-indanon)-2-yl]methyl piperidine hydrochloride of Formula I in Polymorph (I) morphological crystal form.

### BACKGROUND OF THE INVENTION

Donepezil hydrochloride of Formula I is known for its excellent anti-acetyl-cholinesterase activity, and it is an effective active ingredient in pharmaceutical preparations for treatment and prevention of diseases such as Alzheimer disease and senile dementia.

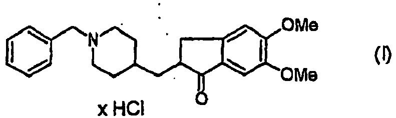

For the preparation of donepezil hydrochloride several methods have been known. Most of them involve the catalytic hydrogenation of an ethylene double bond ("ylide" bond) in the side chain or/and of the pyridine ring. One part of these methods applies hydrogenating after benzylating. According to Example 4 of European Patent No. 296,560 donepezil hydrochloride is obtained with reducing 1-benzyl-4-[(5,6-dimethoxy-1-indanon)-2-ylidenyl]-methyl-piperidine hydrochloride as shown in the next scheme. According to Example 3 of this patent 5,6-dimethoxy-1-indanon is reacted with the complicatedly and costly prepared N-benzyl-pyperidin-4-carbaldehyde to form 1-benzyl-4-[(5,6-dimethoxy-1-indanon)-2-ylidenyl]-methyl-piperidine hydrochloride in an industrially tedious reaction at -70 C° in the presence of butyl lithium.

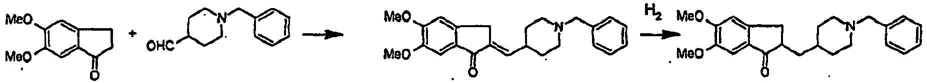

According to another process disclosed in this patent 5,6-dimethoxy-1-indanon is reacted by N-benzoyl-piperidine-4-carbaldehyde and after saturation of the formed "ylide" compound the benzoyl group is removed, and the end-product is obtained by N-benzylation. The common drawback of these procedures are the costly starting materials, the extreme reaction conditions (-70 C°) and the low yield.

The generally used active ingredient form, the donepezil hydrochloride salt, is also revealed in **E**uropean **P**atent No. 296,560**.** The reaction mixture is purified by column chromatography, then the base is solved in dichloromethane, then it is treated with hydrochloric ethyl acetate, followed by evaporating to dry at reduced pressure. The crystalline material is re-crystallized from diisopropyl ether.

In PCT Publications No. WO 97/46,526 and WO 97/46,527 the crystal form of donepezil hydrochloride, obtainable according to the earlier **E**uropean **P**atent No. 296,560**,** was named Polymorph (I), and characterized with the following IR bands obtained in KBr pellet: 463, 502, 563, 589, 604, 701, 750, 759, 799, 860, 922, 947, 972, 1012, 1012,1038, 1104, 1120, 1128, 1175, 1192, 1218, 1250, 1267, 1316, 1368, 1410, 1433, 1440, 1455, 1472, 1502, 1591, 1606, 1644, 1684, 2412, 2530, 2559, 2595, 2620, 2717, 2840, 2858, 2924, 3004, 3074, 3259, 3373, 3547, 3589 cm⁻¹.

The following complicated 9-step synthesis is described in PCT Publication No. WO 97/22,584 that can also be carried out with extra difficulties:

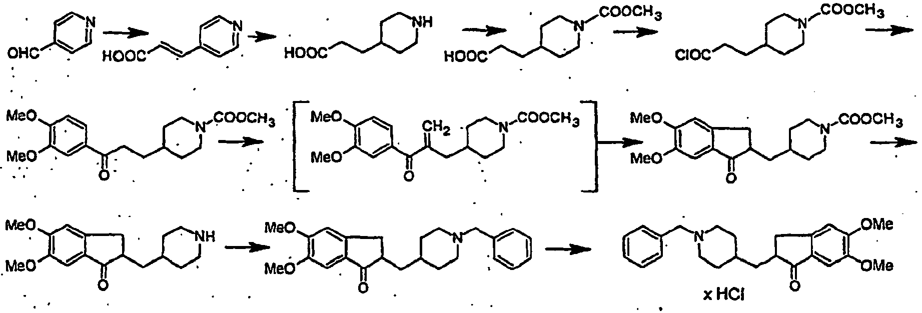

In a part of the synthesis methods an intermediate including saturated side chain and piperidine ring is converted into the end-product.

In PCT Publication No. WO 00/09,483 also a complicated 7-step synthesis is described wherein after benzylation the indole ring is formed by cyclisation of a saturated intermediate. 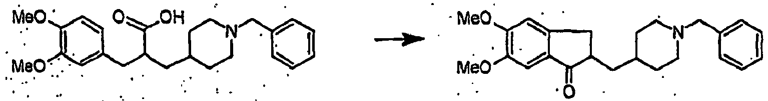 According to **E**uropean **P**atent Application No. EP 1,386,607 donepezil base is obtained by des-ethoxycarbonylation of 1-benzyl-4-[{(5,6-dimethoxy-2-ethoxy carbonyl-indan-1-one)-2-yl}methyl]-piperidine:

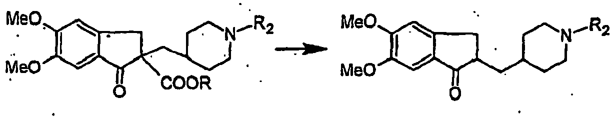

In a similar procedure in PCT Publication No WO 05/03,092 an excess group (Y) is removed also in the last step:

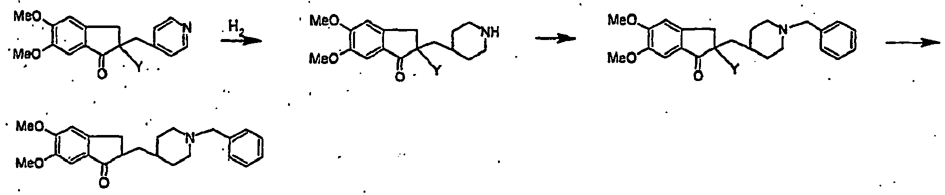

The procedures above can not be taken into account as industrial methods because of their low yield.

The common drawbacks of the following procedures are the high-quantity-use of the costly Adam's catalyst (PtO₂).

In **E**uropean **P**atent Application No. 1,047,674 first 5,6-dimethoxy-2-ethoxycarbonyl-2-[(4-pyridyl)-methyl-]-1-indanon is produced from 5,6-dimethoxy-1-indanon, followed by desethoxycarbonylisation, and then it is transferred to benzyl-halogenide quaternary salt. The end product is obtained by hydrogenation from the quaternary salt:

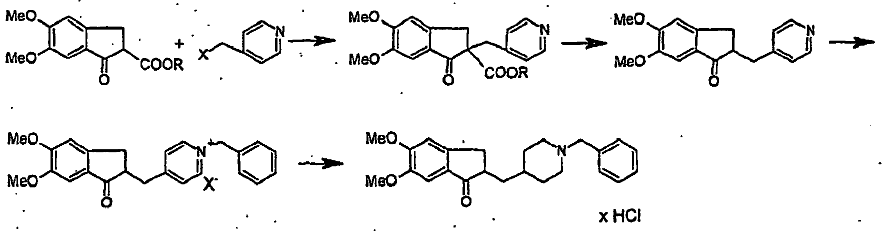

According to the process described in European Patent Application No. 711,756 in a condensation reaction 4-[(5,6-dimethoxy-1-indanon)-2-ylidenyl]-methyl-pyridine is prepared from 5,6-dimethoxy-1-indanon-4-aldehyde, which is quaternized by a benzyl-halogenide. This quaternary salt is hydrogenized during catalytic circumstances:

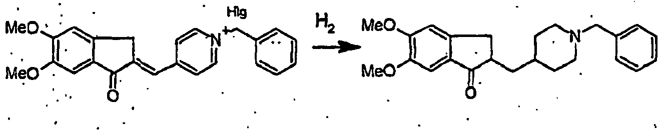

The main disadvantage of the above procedures is the large scale use of the costly catalyst. Our research experiences proved that in these circumstances significant quantities of unwanted debenzylated and over-hydrogenated derivatives (e.g. cyclohexyl one) appear.

In these procedures the purity of the products is not reported and the production methods of the hydrochloride salt are not given.

Also 4-[(5,6-dimethoxy-1-indanon)-2-ylidenyl]-methyl-pyridine is used in U.S. Patent No. 6,649,765**.** Here the starting material is reduced in the presence of 10 %(m/m) Adam's catalyst, then the evolving pyridine derivative is benzylated.

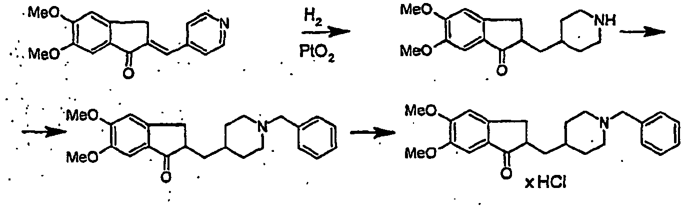

A similar procedure is discovered in PCT Publication No. WO 04/082,685 A1**.** In this procedure the "ylide" bond is hydrogenated with the aid of palladium catalyst, and then 10 % Adam's catalyst is used for the hydrogenation of the pyridine ring:

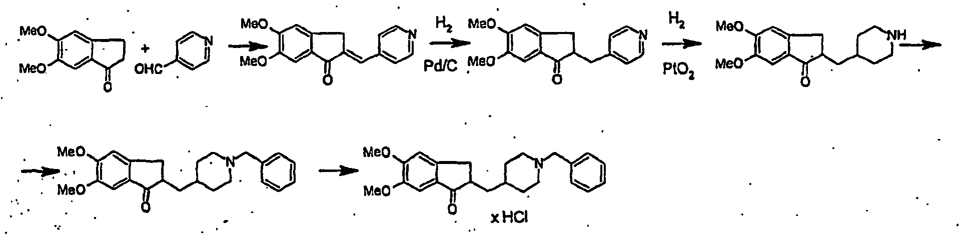

The saturation of the "ylide" double bond in the presence of palladium catalyst is published in Bioorganic & Medicinal Chemistry Letters 2 (2002) 2565-2568, and in Journal of Pharmaceutical and Biomedicinal Analysis 35 (2004) 1047-1058. The latter paper describes that in basic media the 4-[(5,6-dimethoxy-1-indanon)-2-ylidenyl]-methyl-pyridine reduces for four different products from which the impurities of donepezil can be prepared.

In these two applications 4-[(5,6-dimethoxy-1-indanon)-2-ylidenyl]-methyl-pyridine base is reduced. In PCT Publication No. WO 04/082,685 it is mentioned but not detailed that not only the appropriate base can be reduced, but its salts, as well. However, this application does not report on apparent purity differences regarding the evolved products.

In reproduction experiments we also confirmed the establishments of this article of Journal of Pharmaceutical and Biomedicinal Analysis. During reduction of the base, unfortunately, not only the "ylide" double bond and the pyridine ring are saturated, but at least partially the keto group of 5,6-dimethoxy-1-indanon, as well.

According to PCT Publication No. WO 05/076,749 donepezil is obtained in the following reaction scheme:

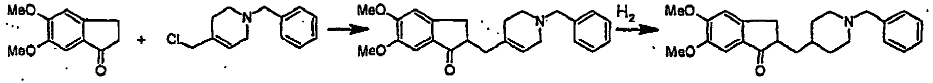

In the process discovered in U.S. Patent Application No. 04/143,121 4-[(5,6-dimethoxy-1-indanon)-2-ylidenyl]-methyl-pyridine is hydrogenated to form 4-[(5,6-dimethoxy-1-indanon)-2-yl]-methyl-piperidine, followed by benzylation to give donepezil. According to our experiences in this way, with the hydrogenation of the base, enough pure donepezil can not be produced on industrial scale.

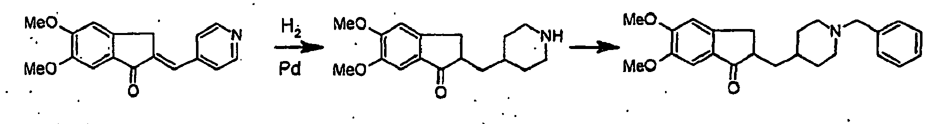

In PCT Publication No. WO 05/044,805 donepezil is prepared according to the following reaction scheme:

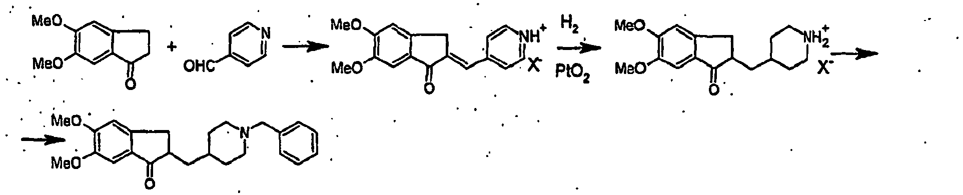

The hydrogenating step is known basically influencing the purity of the end product. Although, in principle, this description determines a wide variety of catalysts, according to Examples 2 and 6 regarding hydrogenation, in the practice it is confined only to the obvious use of PtO₂ catalyst. The application of this catalyst not only makes the procedure too expensive, but it involves the risk that hydrogenating can not be controlled perfectly, and the occurrence of the under- and over-hydrogenated products can not be avoided. The application of p-toluene-sulphonic acidic salt is also a drawback as it appears comparing the results tabulated in Examples 1 and 2 of this paper.

### SUMMARY OF THE INVENTION

The present invention provides a novel, industrially realisable and economically preferable process for production of highly pure 1-benzyl-4-[(5,6-dimethoxy-1-indanon)-2-yl]methyl piperidine hydrochloride *i.e*., donepezil hydrochloride as described in the claims and exemplarily shown in the following reaction scheme, in Polymorph (I) morphological crystal form.

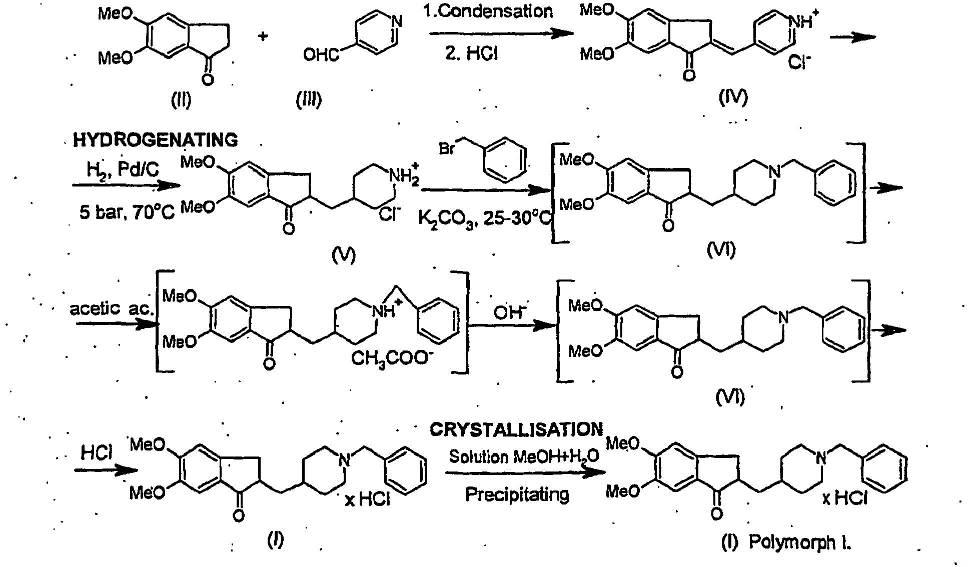

In one of the key steps of the process, during the hydrogenation 5,6-dimethoxy-2-(pyridine-4-ylmethylene)indan-1-one hydrochloride is saturated using Pd carbon to get 4-[(5,6-dimethoxy-1-indanon)-2-yl]-methyl piperidine at more than 97 % HPLC purity. In the crystallization step donepezil-hydrochloride is crystallized from an aqueous alcoholic solvent to get Polymorph (I) in at least 99.95 % HPLC purity.

### DETAILED DESCRIPTION OF THE INVENTION

During our experimental work we found unexpectedly, that if the considerably reaction active 4-[(5,6-dimethoxy-1-indanon)-2-ylidenyl]-methyl-pyridine hydrochloride salt (V) is reduced, in the presence of the less expensive and industrially applicable and less active palladium carbon catalyst, at low temperature, then the wanted crude 4-[(5,6-dimethoxy-1-indanon)-2-yl]-methyl-piperidine (V) can be obtained in one step, in appropriate purity and in a good yield. As the benzyl group is built in a later step, formation of methyl-cyclohexyl over-hydrogenating contaminations can be avoided. In this hydrogenating method more than 99.0% HPLC purity is achieved. The high purity of the early intermediate assures the high purity of the later intermediates, the high purity donepezil hydrochloride salt from which at last the extremely pure donepezil hydrochloride is produced in the wished Polymorph (I) crystal form, which end-product is practically free from every solvent residue.

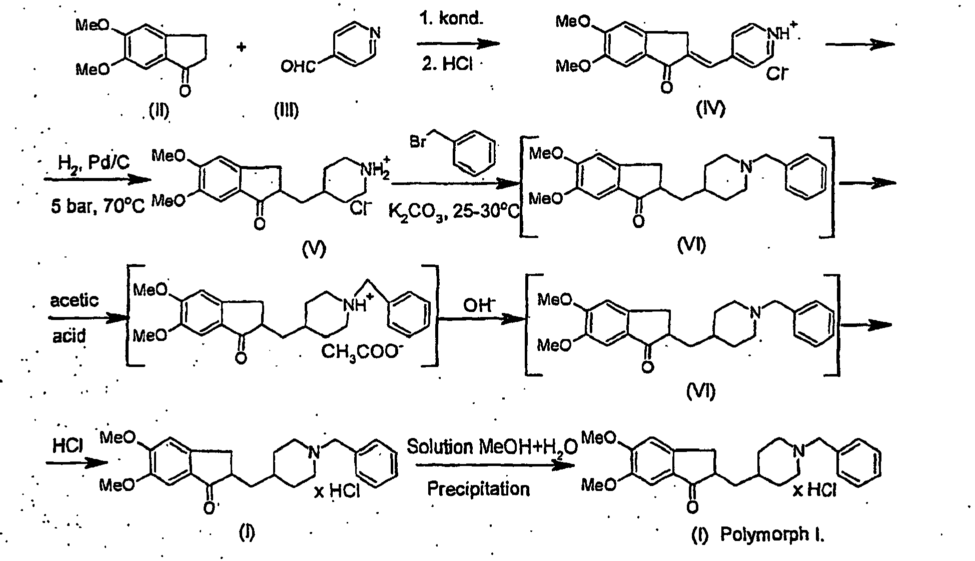

Consequently, the high purity of the product can be assured according to the above reaction scheme in which 5,6-dimethoxy-1-indanon (II) and pyridine-4-aldehyde(III) are reacted in a condensation reaction to get 4-[(5,6-dimethoxy-1-indanon)-2-ylidenyl]-methyl-pyridine base, then the hydrochloric acid salt of the latter (IV) is hydrogenated in acetic acid, at 50-70°C temperature and 5 bar pressure to get 4-[(5,6-dimethoxy-1-indanon)-2-yl]-methyl-piperidine (V). From this intermediate with known manner, with benzylation, followed by salt-forming including phase exchanges for further purification, donepezil hydrochloride is produced. From this material, after an aqueous alcoholic solving and deposition, the extra pure Polymorph (I) donepezil hydrochloride is obtained, practically free from solvent residues. We recognized that such a highly pure end-product can only be obtained if all of the consecutive steps are determined appropriately and especially in the two key steps, in the hydrogenation and crystallization, surprisingly better methods are used than earlier.

The first description of Polymorph (I) of donepezil hydrochloride salt can be found in **E**uropean **P**atent No. 296,560**,** according to which it is formed with purifying a reaction mixture by column chromatography, the base is solved in dichloromethane, then the solved donepezil base is treated by hydrochloric acidic ethyl acetate followed by evaporating in vacuum. The crystalline material is re-crystallized using methanol and isopropyl ether.

Later, in the PCT Publications No.'s WO 97/46,526 and WO 97/46,527 (European Patent No.'s 1,019,374 and 1,211,243**)** this modification was named Polymorph (I) that was characterized by the absorption bands of infra red spectra of the material obtained from KBr pellets. Characterization of the new Forms II, III, IV and V were also described in these documents. U.S. Patent No. 6,140,321 describes Polymorph (III). The PCT application No. WO 04/87,660 provides a preparation method for the amorphous modification.

According to the teaching of PCT Publication No. WO 97/46,527 (and European Patent No.'s. 1,019,374 and 1,211,243**)** Polymorph (I) of donepezil hydrochloride can be prepared in laboratory circumstances.

In one of the preferred processes (No. 1-7), donepezil base is solved in a low carbon number alcohol, salt is formed by hydrochloric acid or hydrogen chloride, then the wanted polymorph (I) is precipitated by t-butyl-methyl-ether (Example 29) or by diisopropyl-ether (Example 30) or by ethyl acetate (Example 31). From the untreatable slurry Polymorph (I) can be filtered poorly. In industrial scale the method can not be applied. In another preferred process (No. 1-9) Polymorph (I) donepezil hydrochloride is prepared by re-crystallisation. Donepezil hydrochloride is solved in a low carbon number alcohol (advantageously in methanol), then its solubility is decreased by different precipitating agents such as t-butyl-methyl-ether (Example 39) or ethyl acetate (Example 40) or n-hexane (Example 41), and the crystalline material is filtered and dried. In all of these descriptions the precipitating agent is added to the solution of donepezil or donepezil-hydrochloride. This description does not report on solvent residue data of donepezil-hydrochloride (I).

It is notable that in these examples on the one hand the applied solvents are not suitable for large scale production (flash-point of diethyl-ether is 40°C), and on the other their use is permitted only in extremely justified cases (as in case of diisopropyl-ether) in the pharmaceutical industry. It is also known from literature (PCT Publication No. WO 1997/46527**)** that the methyl tert-butyl ether is also suitable for producing Polymorph (I) donepezil hydrochloride. This description does not report on the reversed addition, and does not deal with the solvent residue.

According to our investigations from large number of experimental data we concluded that independently of the addition order (diethyl ether is added into the methanolic donepezil hydrochloride solution or contrary) Polymorph (III) can crystallize out instead of the wanted Polymorph (I). Economical industrial procedures can not be based on such an uncertain technology. In the known procedures Polymorph (I), which contains almost one mol bounded water, is crystallized out from water-free solvents. During experiments it was learned that at 60-80 °C donepezil hydrochloride can lose this water, but at room temperature and 40-60 % relative humidity -it takes the water rapidly back from the air. We concluded that according to the known procedures first an unstable anhydrous transient modification of Polymorph (I) is produced, and during processing this transient form changes into the stable water-containing Polymorph (I) crystal modification. In large scale processing this uncontrolled forming is not admissible, because in the pharmaceutical production the good reproducibility is a basic requirement.

Surprisingly, it was found that the solvent residue concentration of the product strongly depends on the addition order of the components. So if the anhydrous methanolic solution of donepezil hydrochloride was added dropwise into the methyl tert-butyl ether solvent containing Polymorph (I) seeds, then the methyl tert-butyl ether residue of the product was about 2500 ppm. In contrary, when the methyl tert-butyl ether was added dropwise into the anhydrous methanolic donepezil hydrochloride solvent containing seeds then the residue of methyl tert-butyl ether was about 6500 ppm.

According to our investigations the water content of the applied alcohol can be increased further to a certain limit without the problem of forming Polymorph (IV), as could be expected according to EP 1211243**.** Polymorph (1) with the necessary one mol water content is evolving if the quantity of water in the solution is 2 - 20 times more than the theoretical one mol. We experienced surprisingly, that the solvent residue data are strongly dependent on the water content of the alcohol used. We stated that if donepezil hydrochloride is solved in methanol containing 2-18% (v/v), advantageously containing 4% (v/v) water, then the contaminating solvent residue will be an order of magnitude less; only 200-300 ppm. The crystallization of Polymorph (I) is advantageously assured with Polymorph (I) seeds suspended in the precipitant solvent, which poorly solves donepezil hydrochloride.

Our further investigations disclosed that forming of Polymorph (I) requires the appropriate adjusting of temperature. Independently of the addition order of components, above 20°C Polymorph (III) crystallizes, decreasing temperature facilitates forming of Polymorph (I). However, at a too low temperature the quantity of the solvent residue increases.

### EXAMPLES

### Example 1.: Reproduction experiment for preparing 2-(4-piperidinylmethyl)-5,6-dimethoxy-1-indenon para-toluene-sulphonic acidic salt according to Example 2. in PCT Publication No. WO 2005/044805 A1:

4,02 g 2-(4-pyridylmethylene)-5,6-dimethoxy-1-indenon para-toluene-sulphonic acid salt was dissolved in 300 ml anhydrous methanol, followed by addition of 330 mg PtO₂ catalyst, and the mixture was hydrogenated with stirring at room temperature under atmospheric for 10.5 hours. The solid was filtered off, washed with 50 ml anhydrous methanol. The liquid phase was evaporated to dryness, the residue was dissolved in 150 ml anhydrous isopropanol with warming, then the solution was cooled for crystallization to obtain 2.02 g of the title compound. The mother liquor was evaporated to 15 ml volume to give more 0.46 g material. Combining the two portions 2.86 g of the title compound was obtained.

Results of the HPLC analysis concerning to the contents of the product is shown in the next table, where the results of experiments with longer reaction times are also demonstrated.

| Time of saturation | Debenzyl-donepezil HPLC (%) | D-vinyl ketone HPLC (%) | Pyridylmethyl-indanone HPLC (%) | Others HPLC (%) |
|---|---|---|---|---|
| | 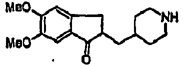 | 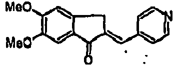 | 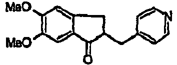 | |
| 7^{h} | 52.46 | 5.54 | 25.95 | 16.05 |
| 10.5^{h} | 57.38 | 5.50 | 20.43 | 16.69 |
| prepared after 7^{h} reaction | 64.42 | 6.34 | 10.25 | 18.99 |

### Example 2: Reproduction experiment for preparing 2-(4-piperidinylmethyl)-5,6-dimethoxy-1-indenone HCl salt according to Example 2. in PCT Publication No. WO 2005/044805 A1:

3.17 g 2-(4-pyridylmethylene)-5,6-dimethoxy-1-indenone hydrochloric acidic salt was dissolved in 300 ml anhydrous methanol, followed by addition of 330 mg PtO₂ catalyst; and the mixture was hydrogenated with stirring at room temperature under atmospheric for 10.5 hours. The solid was filtered off, washed with 50 ml anhydrous methanol. The liquid phase was evaporated to dryness, the residue was dissolved in 150 ml anhydrous isopropanol with warming, then the solution was cooled to 0°C temperature for crystallization to obtain 2.02 g of the title compound. The mother liquor was evaporated to 15 ml volume to give more 1.11 g material. Combining the two portions 3.13 g of the title compound was obtained.

In the next table the results of the HPLC analysis concerning the contents of the product are shown. The results of experiments with longer reaction times are also demonstrated.

| Time of saturation | Debenzyl-donepezil HPLC (%) | D-vinyl ketone HPLC (%) | Pyridylmethyl-indanone HPLC (%) | Others HPLC (%) |
|---|---|---|---|---|
| | 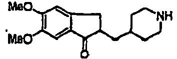 | 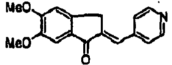 | 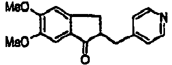 | |
| 7^{h} | 68.56 | 8.50 | 9.41 | 13.53 |
| 10.5^{h} | 74.53 | 8.48 | 1.89 | 15.10 |
| prepared after 7^{h} reaction | 75.28 | 9.38 | 0.99 | 14.35 |

**Example 3.:** 200 l acetic acid, 2.2 kg charcoal containing 10 % palladium suspended in 22 l acetic acid, and 22.24 kg 4-[(5,6-dimethoxy-1-indanon)-2-ylidenyl]-methyl-pyridine hydrochloride (IV) were measured into a 500 1 inertized hydrogenating autoclave, and the mixture was hydrogenated at 68 - 72 °C, under 5 atm overpressure, with intensive stirring, until decreasing of the pressure came to an end. The autoclave was cooled to 20 - 25 °C, the catalyst was filtered off. The filtrate was concentrated in vacuum to 66 1 volume then under stirring 72 l methyl-isobutyl-ketone was added dropwise into it. The crystalline material was filtered off, and washed with methyl-isobutyl-ketone. The moist material was solved in 210 l boiling methanol, and then it was cooled to 0-5 °C. The crystalline material was filtered off, washed, and after drying 15.12 kg 4-[(5,6-dimethoxy-1-indanon)-2-yl]-methyl-piperidine (V) was obtained.

Results of the TLC analysis concerning to the contents of the product is shown in the next table:

| Time of saturation | Debenzyl-donepezil (%) | Desoxo-debenzyl-donepezil (%) | D-vinyl ketone (%) | Pyridylmethyl-indanone (%) |
|---|---|---|---|---|
| | 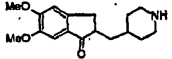 | 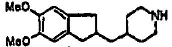 | 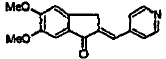 | 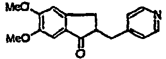 |
| 7^{h} | | Under the detection limit (TLC) | Under the detection limit (TLC) | <1% |
| prepared after 7^{h} reaction | 99.4 | 0.5 | Under detec-tion limit (HPLC) | Under detection limit (HPLC) |

**Example 4.:** 500 l acetic acid, 32.58 kg 4-[(5,6-dimethoxy-1-indanon)-2-yl]-methylpiperidine (V), 27.60 kg potassium-carbonate, 6.5 l water and 2.2 kg charcoal containing 10 % palladium and suspending in 22 l acetic acid were measured into a 1000 l volume autoclave, then a solution of 12.5 l benzyl-bromide in 40 l ethyl acetate were added dropwise into it. The reaction mixture was stirred intensively at 25 - 30 °C for 4 hours. After checking accomplishment of the reaction, the solid was filtered off. 300 l water and 12 l acetic acid was added to the filtrate and after stirring the phases were separated. The organic layer was washed with 50 I water, the phases were separated again. The product was in the united aqueous phase, to which 200 1 ethyl acetate was added and 100 1 aqueous solution of 10.0 kg. NaOH was added dropwise into it. After stirring the phases were separated, the aqueous phase was washed with a new portion of 1001 ethyl acetate and the organic phase was washed with water. The separated organic phase was dried on anhydrous sodium-sulphate, and filtered. The filtrate was concentrated to 50 in vacuum, 50 l methanol was added, homogenized, and evaporated, then new 50 l methanol was added, and evaporated again.

With stirring the residue was solved in 140 l methanol and cooled to 5 - 10 °C. 38 l hydrochloric acidic methanol (a mixture of 10.46 kg cc hydrochloric acid and 23.75 kg methanol) was added dropwise into it. The crystallization was made completed with dropping 280 l methyl tert-butyl ether containing Polymorph (I) seeds. The product was filtered off, washed twice with 35 l methyl tert-butyl ether, and dried at 35 - 40 °C in the air to obtain 30.7 kg Polymorph (I) donepezil hydrochloride active ingredient. Its analytical data are shown in the following table:

| | Donepezil HPLC (%) | Desoxo-donepezil HPLC (%) | Debenzyl-donepezil HPLC (%) | Others HPLC (%) |
|---|---|---|---|---|
| | 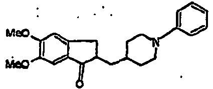 | 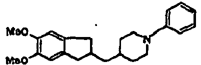 | 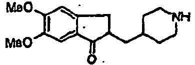 | |
| prepared after 7^{h} reaction | 99,97 | Under detection limit | <0,03 | Under detection limit |

**Example 5.:** 34.5 1 methanol, 1.5 l demineralized water, 9.00 kg donepezil hydrochloride (in principle any polymorph modification is suitable) were measured into a 150 1 volume autoclave equipped with stirrer and partial condenser. When the temperature of suspension was elevated to 50-55°C the solid dissolved again. The solution was cooled to 25-30°C, and filtered to fiber-free. 90 l methyl tert-butyl ether and 90 g Polymorph (I) donepezil hydrochloride were measured into a 150 1 volume autoclave, temperature of the suspension was decreased to among 5-10°C, and under a continuous intensive stirring the donepezil hydrochloride solution was added uniformly in a 30 minute period. After a new 30 minute's stirring the crystallized solid was filtered off and washed with 9 l methyl tert-butyl ether. The crystalline material was dried at 35-40° to obtain 8.7 kg donepezil hydrochloride in Polymorph (I) containing low solvent residue. The characteristic concentration of methyl tert-butyl ether residue was 150-750 ppm, while the other contaminating solvent residue components were under the detection limit.

## Claims

1. A process for the preparation of Polymorph (I) donepezil hydrochloride in at least 99,95% HPLC purity of Formula **I** with 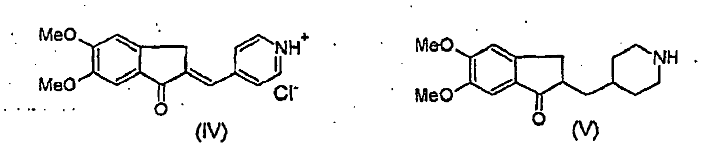 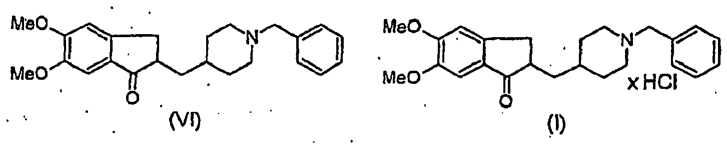 hydrogenating of 5,6-dimethoxy-2-(pyridine-4-ylmethylene)indane-1-one salt
intermediate of Formula **IV**, and subsequent
benxylating of the obtained 4-[(5,6-dimethoxy-1-indanon)-2-yl]-methyl piperidine intermediate of Formula **V**, and subsequent
salt-forming steps with reacting donepezil base of Formula **VI**; and subsequent re-crystallizing of the obtained donepezil hydrochloride of Formula I from an aqueous alcoholic solvent,
which process comprises:
a. accomplishing the said hydrogenating of 5,6-dimethoxy-2-(pyridine-4-yl methylene)indan-1-one hydrochloride of Formula **IV** in the presence of charcoal Pd catalyst, and
b. accomplishing the said salt-forming steps with reacting donepezil base of Formula **VI** by acetic acid, then extracting the acetic acidic salt into aqueous phase, then transforming it again to donepezil base in the presence of a base, then transforming the latter into donepezil hydrochloride of Formula I in the presence of hydrogen chloride, and
c. accomplishing the said re-crystallizing with precipitating the Polymorph (I) donepezil hydrochloride product from aqueous alcoholic solution containing 2-18% (v/v) water at a temperature of 20°C or lower.

2. The process of claim 1 wherein the said hydrogenating comprises the application of charcoal containing 5-15 % palladium as a catalyst.

3. The process of claim 2 wherein the said hydrogenating further comprises the application of 4-6 atm. overpressure and temperature at 60-80 °C.

4. The process of claim 1 wherein the said salt-forming steps comprise reacting the donepezil base by acetic acid in ethyl acetate solution and transferring donepezil acetate into aqueous phase.

5. The process of claim 1 wherein the said re-crystallizing comprises resolving donepezil hydrochloride in aqueous methanol containing 3-5 %(v/v) water.

6. The process of claim 1 wherein the said re-crystallizing comprises that the aqueous methanolic solution of donepezil hydrochloride is added to the methyl-tert-butyl-ether precipitating agent containing Polymorph (I) seeds.

## Patentansprüche

1. Ein Verfahren zur Herstellung von Donepezilhydrochlorid Polymorph (I) der Formel I mit einer HPLC-Reinheit von mindestens 99.95 % 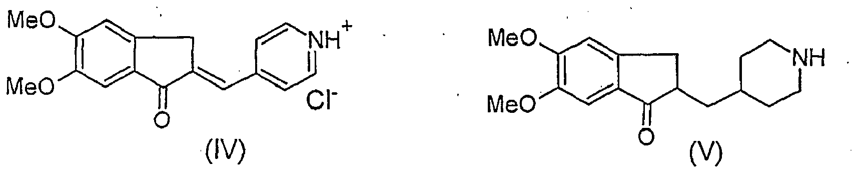 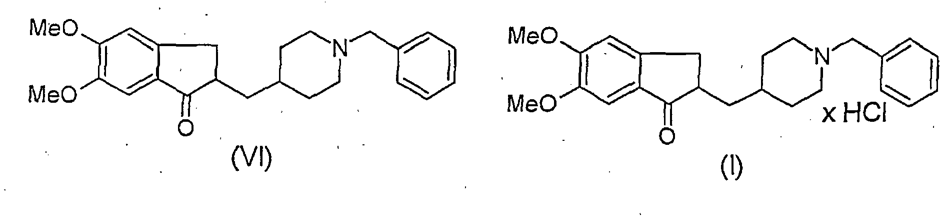 durch Hydrieren des Zwischenproduktes 5,6-Dimethoxy-2-(pyridin-4-ylmethylen)indan-1-onsalz der Formel IV und anschließendes
Benzylieren des erhaltenen Zwischenproduktes 4-[(5,6-Dimethoxy-1-indanon)-2-yl]-methylpiperidin der Formel V und anschließende
salzbildende Schritte durch Umsetzen der Donepezilbase der Formel VI und anschließendes
Rekristallisieren des erhaltenen Donepezilhydrochlorids der Formel I aus einem wässrigen alkoholischen Lösungsmittel,
wobei das Verfahren umfasst:
a. Durchführen der Hydrierung von 5,6-Dimethoxy-2-(pyridin-4-ylmethylen)indan-1-onhydrochlorid der Formel IV in Gegenwart eines Aktivkohle-Pd-Katalysators und
b. Durchführen der salzbildenden Schritte durch Umsetzen der Donepezilbase der Formel VI mit Essigsäure, dann Extrahieren des Essigsäuresalzes in eine wässrige Phase, dann erneutes Umwandeln des Salzes in die Donepezilbase in Gegenwart einer Base,
dann Umwandeln des Letzteren in ein Donepezilhydrochlorid der Formel I in Gegenwart von Wasserstoffchlorid und
c. Durchführen der Rekristallisation durch Ausfällen des Donepezilhydrochlorid Polymorph (I)-Produktes aus einer wässrigen alkoholischen Lösung, enthaltend 2-18 Vol.-% Wasser, bei einer Temperatur von 20 °C oder weniger.

2. Das Verfahren nach Anspruch 1, wobei die Hydrierung die Anwendung von Aktivkohle, enthaltend 5-15 % Palladium, als einen Katalysator umfasst.

3. Das Verfahren nach Anspruch 2, wobei die Hydrierung ferner die Anwendung von 4-6 atm Überdruck und einer Temperatur von 60-80 °C umfasst.

4. Das Verfahren nach Anspruch 1, wobei die salzbildenden Schritte das Umsetzen der Donepezilbase mit Essigsäure in Ethylacetatlösung und das Überführen von Donepezilacetat in eine wässrige Phase umfasst.

5. Das Verfahren nach Anspruch 1, wobei die Rekristallisierung das Lösen von Donepezilhydrochlorid in wässrigem Methanol, enthaltend 3-5 Vol.-% Wasser, umfasst.

6. Das Verfahren nach Anspruch 1, wobei die Rekristallisierung das Zugeben der wässrigen methanolischen Lösung von Donepezilhydrochlorid zum Methyl-tert-butylether-Fällungsmittel, enthaltend Polymorph (I)-Keime, umfasst.

## Revendications

1. Méthode de préparation d'un Polymorph (I) de chlorhydrate de donépézil de formule (I) avec une pureté HPLC d'au moins 99,95% avec 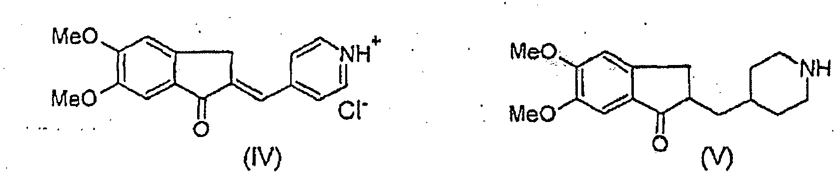 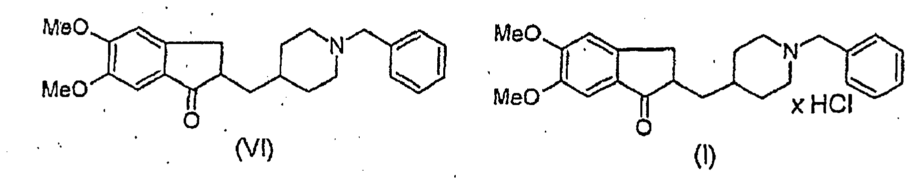 hydrogénation de l'intermédiaire sel de 5,6-diméthoxy-2-(pyridine-4-ylméthylène)indane-1-one de formule IV, et ensuite
benzylation de l'intermédiaire 4-[(5,6-diméthoxy-1-indanon)-2-yl]-méthyl pipéridine de formule V obtenu, et ensuite
des étapes de formation de sel par réaction de la base donépézil de formule VI, et ensuite recristallisation du chlorhydrate de donépézil de formule I obtenu à partir d'un solvant hydroalcoolique, ladite méthode comprenant :
a. l'accomplissement de ladite hydrogénation du chlorhydrate de 5,6-diméthoxy-2-(pyridine-4-ylméthylène)indan-1-one de formule IV en présence d'un catalyseur de Pd sur charbon, et
b. l'accomplissement de la dite étape de formation de sel par réaction de la base donépézil de formule VI avec de l'acide acétique, puis par extraction du sel d'acide acétique dans une phase aqueuse, puis par sa transformation en base donépézil en présence d'une base, puis par transformation de cette dernière en chlorydrate de donépézil de formule I en présence de chlorure d'hydrogène, et
c. l'accomplissement de ladite étape de recristallisation par précipitation du Polymorphe (I) de chlorhydrate de donépézil produit à partir d'une solution hydroalcoolique contenant 2-18 % (v/v) d'eau, à une température de 20°C ou moins.

2. Méthode selon la revendication 1 dans laquelle ladite hydrogénation comprend l'application d'un charbon contenant de 5-15% de palladium en tant que catalyseur.

3. Méthode selon la revendication 2 dans laquelle ladite hydrogénation comprend de plus l'application d'une surpression de 4-6 atm et une température de 60-80°C.

4. Méthode selon la revendication 1 dans laquelle lesdites étapes de formation de sel comprennent la réaction de la base donépézil avec de l'acide acétique dans une solution d'acétate d'éthyle et le transfert de l'acétate de donépézil en phase aqueuse.

5. Méthode selon la revendication 1 dans laquelle ladite recristallisation comprend la résolution du chlorhydrate de donépézil dans du méthanol aqueux comprenant 3-5% (v/v) d'eau.

6. Méthode selon la revendication 1 dans laquelle ladite recristallisation comprend l'addition de la solution aqueuse méthanolique de chlorhydrate de donépézil dans l'agent de précipitation méthyl-tert-butyl-éther contenant des germes de Polymorphe (I).
